# EUROPÄISCHE PATENTSCHRIFT

(11) **EP 3 417 898 B1**
(45) Veröffentlichungstag und Bekanntmachung des Hinweises auf die Patenterteilung: **07.10.2020**
(21) Anmeldenummer: 18000527.4
(22) Anmeldetag: 15.06.2018
(51) Int. Cl.: A61M 16/06

(54) **ATEMMASKE MIT KOPPLUNGSELEMENT FÜR EINE BÄNDERUNG**
RESPIRATORY MASK WITH A COUPLING ELEMENT FOR A HEAD HARNESS
MASQUE RESPIRATOIRE POURVU D'ÉLÉMENT DE COUPLAGE POUR UN AGENCEMENT DE BRIDES

(30) Priorität: 19.06.2017 DE 102017005691; 19.06.2017 DE 102017005704
(43) Veröffentlichungstag der Anmeldung: 26.12.2018
(73) Patentinhaber: Löwenstein Medical Technology S.A., 2557 Luxembourg (LU)
(72) Erfinder: Frerichs, Arnold, 21614 Buxtehude (DE); Bechtel, Martin, 21423 Winsen / Luhe (DE); Eifler, Martin, 25348 Glückstadt (DE); Gardein, Joachim, 38430 Icod de Ios Vinos Tenerife/Islas Canarias (ES)
(74) Vertreter: Marx, Thomas

(56) Entgegenhaltungen:
- EP-A1- 2 818 196
- EP-A1- 2 954 920
- EP-A1- 3 375 476
- EP-A2- 2 732 839
- WO-A1-00/78384
- WO-A1-2016/139623
- US-A1- 2004 182 398
- US-A1- 2005 011 521

## Beschreibung

Die Erfindung betrifft ein Patienteninterface mit einem Kopplungselement für eine Bänderung. Für die Befestigung eines Patienteninterface (PI) an einem Patienten oder Benutzer wird meist eine Bänderung verwendet. Das PI weist daher Kopplungselemente für die Bänderung auf. Bei einem PI welches als Beatmungsmaske mit einer Stirnabstützung ausgebildet ist, befinden sich üblicherweise zwei Kopplungselemente an der Stirnabstützung und zwei Kopplungselemente an dem Maskenkörper. Die Verbindung von Kopplungselement und Bänderung kann unter Vermittlung von zusätzlichen Klips oder Adaptern erfolgen. Alternativ kann die Bänderung Schlaufen ausbilden, die unmittelbar an dem Kopplungselement befestigt werden. Die unterschiedlichen Verbindungen von Kopplungselement und Bänderung sind beispielsweise in der EP 2727620 A1 oder der US 20040045551 A1 beschrieben.

Die bekannten Lösungen weisen für ein gegebenes Bänderungsende immer nur ein definiertes Kopplungselement auf. Durch diese feste Zuordnung ist eine individuelle Anpassung für den Patienten meist nicht möglich.

Die EP2818196A1, offenbart ein Patienteninterface mit zumindest einem Kopplungselement für eine Bänderung, wobei das Kopplungselement zumindest eine Symmetrieebene aufweist und zumindest vier Aufnahmemittel für ein Bänderungsende einer Bänderung. Die Aufnahmemittel sind in Paaren beidseitig der Symmetrieebene angeordnet. Ein Bänderungsende ist alternativ an dem einen Aufnahmemittel oder an dem anderen Aufnahmemittel auf einer Seite des Kopplungselementes befestigbar. Das eine Aufnahmemittel ist räumlich benachbart zum anderen Aufnahmemittel und näher zu der Symmetrieebene angeordnet ist als das andere Aufnahmemittel.

Es ist daher die Aufgabe der vorliegenden Erfindung ein Patienteninterface mit einem Kopplungselement für eine Bänderung anzugeben, dass eine individuelle Anpassung für den Patienten ermöglicht

Die Erfindung ist in den angehängten Ansprüchen definiert.

Die Aufgabe wird gelöst durch ein Patienteninterface mit einem Kopplungselement für eine Bänderung, wobei das Kopplungselement zumindest eine Symmetrieebene aufweist und zumindest vier Aufnahmemittel für ein Bänderungsende einer Bänderung wobei die Aufnahmemittel in Paaren beidseitig der Symmetrieebene angeordnet sind und ein Bänderungsende alternativ an dem einen Aufnahmemittel oder an dem anderen Aufnahmemittel auf einer Seite des Kopplungselementes befestigt werden kann und das eine Aufnahmemittel räumlich benachbart zum anderen Aufnahmemittel und näher zu der Symmetrieebene angeordnet ist wobei das Kopplungselement zumindest eine Ebene aufweist, die parallel zu der Anlagefläche, nämlich Stirn eines Benutzers, verläuft und wobei das Kopplungselement sich zumindest abschnittsweise abgewinkelt von der Ebene mit einer gerundeten Kontur erstreckt und eine Aussparung definiert und wobei die Aufnahmemittel unterschiedlich weit von der Stirn eines Benutzers entfernt angeordnet sind.

Das Patienteninterface ist auch dadurch gekennzeichnet, dass das Kopplungselement senkrecht zu der Symmetrieebene eine gedachte Ebene aufweist, die parallel zu der Anlagefläche (beispielsweise Stirn) eines Benutzers verläuft.

Das Patienteninterface ist alternativ auch dadurch gekennzeichnet, dass das Kopplungselement sich zumindest abschnittsweise abgewinkelt von der Symmetrieebene entlang der Ebene E mit einer gerundeten Kontur erstreckt.

Das Patienteninterface ist alternativ auch dadurch gekennzeichnet, dass durch die Aufnahmemittel 5 eine Ebene gebildet ist und durch die Aufnahmemittel 6 eine weitere Ebene gebildet wird, wobei die Ebenen voneinander beabstandet sind.

Das Patienteninterface ist alternativ auch dadurch gekennzeichnet, dass durch die Aufnahmemittel 5 eine Ebene gebildet ist und durch die Aufnahmemittel 6 eine weitere Ebene gebildet wird, wobei die Ebenen auf einen mittleren Abschnitt der Aufnahmemittel 5 und 6 bezogen sind.

Das Patienteninterface ist alternativ auch dadurch gekennzeichnet, dass die Ebenen parallel zueinander oder in einem Winkel zueinander verlaufen.

Das Patienteninterface ist alternativ auch dadurch gekennzeichnet, dass durch die Aufnahmemittel 5 und/oder die Aufnahmemittel 6 zumindest abschnittsweise einen gebogenen Konturverlauf aufweisen.

Das Patienteninterface ist alternativ auch dadurch gekennzeichnet, dass der gebogene Konturverlauf einen gleichbleibenden Krümmungsradius aufweist.

Das Patienteninterface ist alternativ auch dadurch gekennzeichnet, dass der gebogene Konturverlauf einen ansteigenden Krümmungsradius aufweist.

Das Patienteninterface ist alternativ auch dadurch gekennzeichnet, dass der gebogene Konturverlauf von Aufnahmemittel 6 von einem mittleren Bereich des Ausnahmemittels ausgehend einen ansteigenden Krümmungsradius aufweist.

Das Patienteninterface ist alternativ auch dadurch gekennzeichnet, dass die die Aufnahmemittel 6 einen gebogenen Konturverlauf aufweisen, wobei die Aüfnahmemittel 6 zumindest abschnittsweise konvex oder konkav verlaufen.

Das Patienteninterface ist alternativ auch dadurch gekennzeichnet, dass die die Aufnahmemittel 5 und/oder 6 einen gebogenen Konturverlauf aufweisen, der zumindest abschnittsweise von der Stirn eines Benutzers weg weist.

Das Patienteninterface ist alternativ auch dadurch gekennzeichnet, dass die die Aufnahmemittel 5 und/oder 6 einen gebogenen Konturverlauf aufweisen, sodass die die Aufnahmemittel 5 und/oder 6 nur abschnittsweise, beispielsweise im mittleren Abschnitt, an der Stirn eines Benutzers anliegen.

Das Patienteninterface ist ergänzend auch dadurch gekennzeichnet, dass die beiden Aufnahmemittel geometrisch unterschiedlich ausgebildet sind.

Das Patienteninterface ist erfindungsgemäß auch dadurch gekennzeichnet, dass die Aufnahmemittel als geschlossener und geschlitzter Steg ausgebildet sind.

Das Patienteninterface ist erfindungsgemäß auch dadurch gekennzeichnet, dass das Kopplungselement einstückig als Teil einer Stirnabstützung eines Patienteninterface ausgebildet ist und die Symmetrieebene S mit der Längsachse der Stirnabstützung zusammenfällt.

Das Patienteninterface ist erfindungsgemäß dadurch gekennzeichnet, dass die geschlitzten Stegen 6 außen und die geschlossenen Stege 5 relativ zu den geschlitzten Stegen weiter innen, näher an der Symmetrieebene S angeordnet sind.

Das Patienteninterface ist alternativ oder ergänzend auch dadurch gekennzeichnet, dass die geschlitzten Stegen und die geschlossenen Stege relativ zu der Symmetrieebene S unsymmetrisch angeordnet sind.

Das Patienteninterface ist alternativ oder ergänzend auch dadurch gekennzeichnet, dass die geschlitzten Stege und die geschlossenen Stege zumindest abschnittsweise parallel zueinander verlaufen.

Das Patienteninterface ist alternativ oder ergänzend auch dadurch gekennzeichnet, dass die geschlitzten Stege und/oder die geschlossenen Stege zumindest abschnittsweise windschief zueinander und/oder zur Symmetrieebene S verlaufen.

Das Patienteninterface ist alternativ oder ergänzend auch dadurch gekennzeichnet, dass die Aufnahmemittel unterschiedlich weit von der Ebene E entfernt angeordnet sind.

Das Patienteninterface ist alternativ oder ergänzend auch dadurch gekennzeichnet, dass die Stege im Querschnitt gerundet oder eckig sind.

Das Patienteninterface ist alternativ oder ergänzend auch dadurch gekennzeichnet, dass die Stege zumindest abschnittsweise eine raue Oberfläche aufweisen.

Das Patienteninterface ist alternativ oder ergänzend auch dadurch gekennzeichnet, dass die geschlitzten Stege andere Oberflächen aufweisen, als die geschlossenen Stege.

Das Patienteninterface ist alternativ oder ergänzend auch dadurch gekennzeichnet, dass die Stege Haltemittel für die Bänderung aufweisen.

Das Patienteninterface ist alternativ oder ergänzend auch dadurch gekennzeichnet, dass ein geschlitzter Steg zwischen den beiden Teilstegen jeweils eine Öffnung für die Bänderung bereitstellt und Öffnung so dimensioniert ist, dass die Bänderung mit ihrer flachen Seite hindurchgefädelt werden kann. Das Patienteninterface ist alternativ oder ergänzend auch dadurch gekennzeichnet, dass die beiden Teilstege mit einem Versatz zueinander angeordnet sind.

Das Patienteninterface ist alternativ oder ergänzend auch dadurch gekennzeichnet, dass die Teilstege unterschiedlich lang sind.

Das Patienteninterface ist alternativ oder ergänzend auch dadurch gekennzeichnet, dass die Teilstege benachbart zur Öffnung Sicherungselemente aufweisen, die ein hinausrutschen der Bänderungsschlaufe aus der Öffnung verhindern.

Die Begriffe Patienteninterface und Atemmaske werden synonym verwendet. Im Sinne der Erfindung ist jedes Interface für die Beatmung umfasst.

So kann das Kopplungselement 1, zum Beispiel in Form einer Lasche, aus demselben biegsamen Material wie das PI hergestellt sein. Infolgedessen ist es zum Beispiel bevorzugt, wenn das Kopplungselement mit dem PI einstückig ausgebildet ist oder zweistückig mit dem Grundkörper des PI über eine Schnittstelle gekoppelt ist. Das Kopplungselement und das PI sind bevorzugt aus Polycarbonat, Polyethylen oder ähnlichen Kunststoffen im Spritzgussverfahren hergestellt.

Das Kopplungselement kann ein Polster aufweisen, welches der Abstützung an der Haut eines Benutzers dient.

Das Kopplungselement kann sich auch unmittelbar an der Haut eines Benutzers abstützen.

Das Kopplungselement kann auch so gestaltet sein, dass ein Aufnahmemittel 5, 6 mit einem Bänderungsende 4 zumindest teilweise bedeckt ist und daher die Bänderung an der Haut eines Benutzers anliegt.

Das Kopplungselement ist erfindungsgemäß einstückig als Teil einer Stirnabstützung ausgebildet

Das Kopplungselement 1 weist zumindest eine Symmetrieebene S auf. Das Kopplungselement 1 weist senkrecht zu der Symmetrieebene S eine gedachte Ebene E auf, die parallel zu der Anlagefläche (beispielsweise Stirn) eines Benutzers verläuft. Das Kopplungselement 1 erstreckt sich zumindest abschnittsweise abgewinkelt von der Symmetrieebene entlang der Ebene E mit einer gerundeten Kontur. Von der Symmetrieeben beabstandet befinden sich zumindest vier Aufnahmemittel 5, 6 für ein Bänderungsende 4 einer Bänderung 3. Die Aufnahmemittel 5, 6 sind in Paaren beidseitig der Symmetrieebene S angeordnet. Das Aufnahmemittel 5 ist räumlich benachbart zum Aufnahmemittel 6 und näher zu der Symmetrieebene S angeordnet als das Aufnahmemittel 6. Durch den gerundeten Konturverlauf des Kopplungselementes 1 befinden sich die Aufnahmemittel 5, 6 unterschiedlich weit von der Ebene E entfernt. Das Aufnahmemittel 6 ist näher an der Ebene E angeordnet als das Aufnahmemittel 5.

Ein Bänderungsende 4 kann alternativ an dem Aufnahmemittel 5 oder an dem Aufnahmemittel 6 auf einer Seite des Kopplungselementes befestigt werden. Da das Aufnahmemittel 5 riäher zu der Symmetrieebene S angeordnet ist als das Aufnahmemittel 6, kann dadurch eine Feineinstellung der Länge des Bänderungsendes 4 erfolgen. Die Aufnahmemittel 5, 6 befinden sich zudem unterschiedlich weit von der Ebene E entfernt. Dadurch kann eine Feineinstellung des Verlaufes der Bänderung erfolgen.

Das Kopplungselement kann auch einstückig als Teil eines Grundkörpers eines PI ausgebildet sein.

Das Kopplungselement weist zumindest vier Aufnahmemittel 2 für Bänderungsenden 4 einer Bänderung 3 auf. Die Aufnahmemittel 2 sind als geschlossener 5 oder geschlitzter Steg 6 ausgebildet. Die Bänderungsenden 4 werden zur Verbindung mit den Stegen 5, 6 mit sich selbst geschlauft 7 und durch einen Klett 8 fixiert. Die Schlaufen 7 umfassen den geschlossenen oder den geschlitzten Steg zumindest teilweise.

Das Kopplungselement 1 weist eine Symmetrieebene S auf von der die zumindest vier Aufnahmemittel 2 beabstandet sind.

Die Aufnahmemittel 2 können so am Kopplungselement 1 angeordnet sein, dass die geschlossenen Stege 5 außen und die geschlitzten Stegen 6 relativ zu den geschlossenen Stegen weiter innen, näher an der Symmetrieebene S angeordnet sind.

Die Aufnahmemittel 2 können so am Kopplungselement 1 angeordnet sein, dass die geschlitzten Stegen 6 außen und die geschlossenen Stege 5 relativ zu den geschlitzten Stegen weiter innen, näher an der Symmetrieebene S angeordnet sind.

Die Aufnahmemittel 2 können so am Kopplungselement 1 angeordnet sein, dass die geschlitzten Stegen 6 und die geschlossenen Stege 5 relativ zu der Symmetrieebene S unsymmetrisch angeordnet sind. Die Aufnahmemittel 2 können so am Kopplungselement 1 angeordnet sein, dass die geschlitzten Stegen 6 und die geschlossenen Stege 5 relativ zu der Symmetrieebene S symmetrisch angeordnet sind.

Fig. 2 Die Aufnahmemittel 2 können so am Kopplungselement 1 angeordnet sein, dass die geschlitzten Stege 6 und die geschlossenen Stege 5 zumindest abschnittsweise parallel zueinander verlaufen.

Die Aufnahmemittel 2 können so am Kopplungselement 1 angeordnet sein, dass die geschlitzten Stege 6 und die geschlossenen Stege 5 zumindest abschnittsweise eine Parallelverschiebung aufweisen.

Fig. 3 - 5 Die Aufnahmemittel 2 sind erfindungsgemäß so am Kopplungselement 1 angeordnet , dass die geschlitzten Stege 6 und/oder die geschlossenen Stege 5 verlaufen.

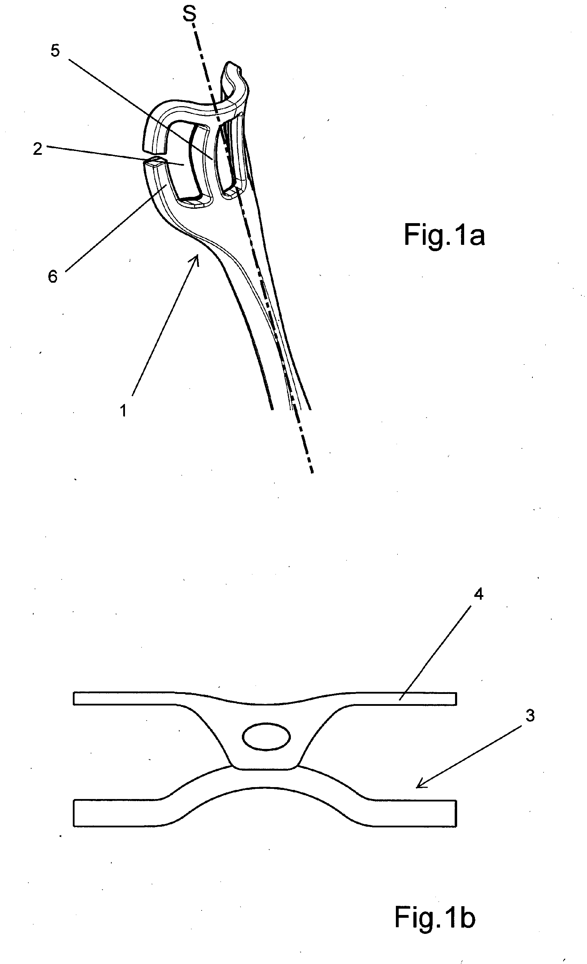

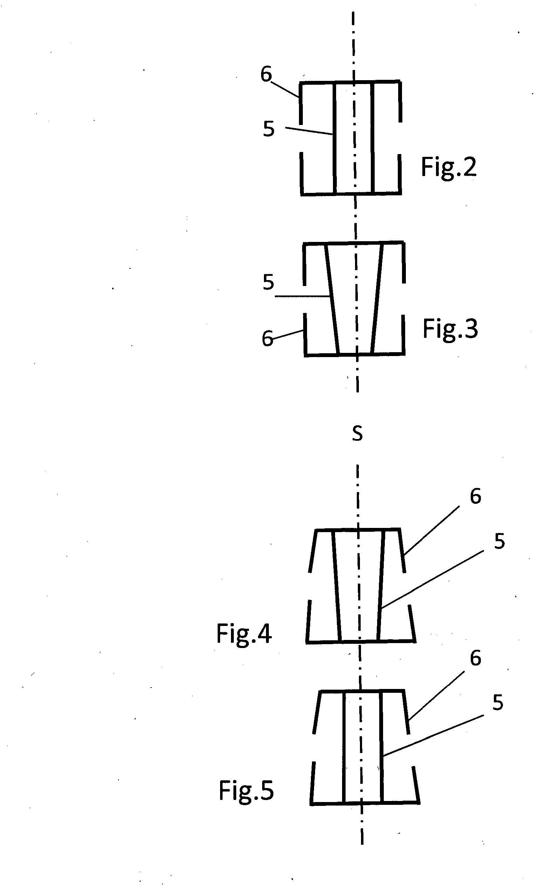

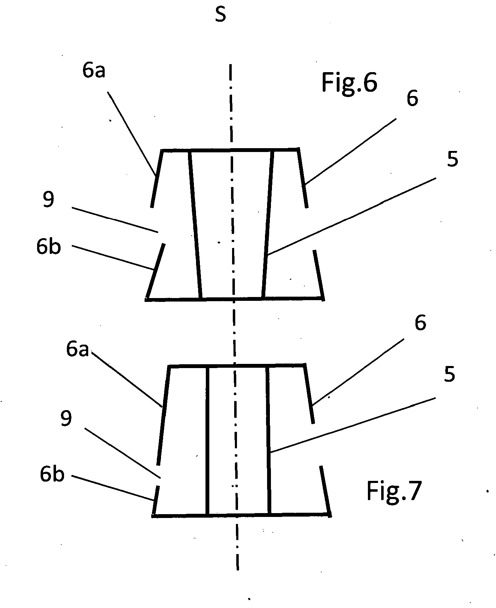

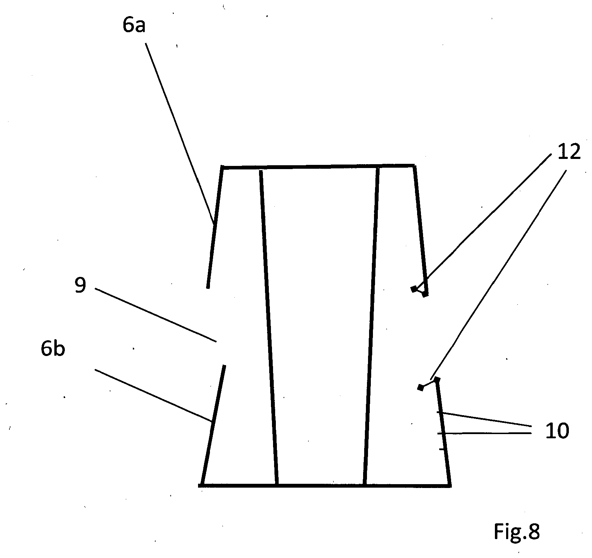

In Fig. 3 sind die die geschlitzten Stege 6 parallel zueinander und die geschlossenen Stege 5 windschief.

In Fig. 4 sind die die geschlitzten Stege 6 zueinander und zu den geschlossenen Stegen 5 windschief. Ebenso sind die geschlossenen Stegen 5 zueinander und zu den geschlitzten Stege 6 windschief

In Fig. 5 sind die geschlossenen Stege 5 zueinander parallel und die geschlitzten Stege 6 zueinander windschief.

Die Stege 5,6 können im Querschnitt gerundet oder eckig sein. Die Stege können beispielsweise auch eine glatte Oberfläche aufweisen. Alternativ ist auch vorgesehen, dass die Stege 5,6 zumindest abschnittsweise eine raue Oberfläche aufweisen.

Die Stege 5,6 können auch Haltemittel 10 für die Bänderung aufweisen. Die Haltemittel sind beispielsweise Haken oder Dornen die sich nur wenig über die Oberfläche erheben.

Ein geschlitzter Steg 6 bietet zwischen den beiden Teilstegen 6a, 6b jeweils eine Öffnung 9 für die Bänderung. Die Öffnung ist bevorzugt so dimensioniert, dass die Bänderung mit ihrer flachen Seite hindurchgefädelt werden kann.

Die geschlitzten Stege 6 können andere Oberflächen aufweisen, als die geschlossenen Stege 5.

Fig. 6
Die geschlitzten Stege 6 können fluchtend oder mit einem Versatz zueinander angeordnet sein

Fig. 7
Die geschlitzten Stege 6 können gleichlange oder unterschiedlich lange Teilstege aufweisen.

Fig. 8
Die Teilstege können benachbart zur Öffnung 9 Sicherungselemente 12 aufweisen, die ein Hinausrutschen der Bänderungsschlaufe aus der Öffnung verhindern.

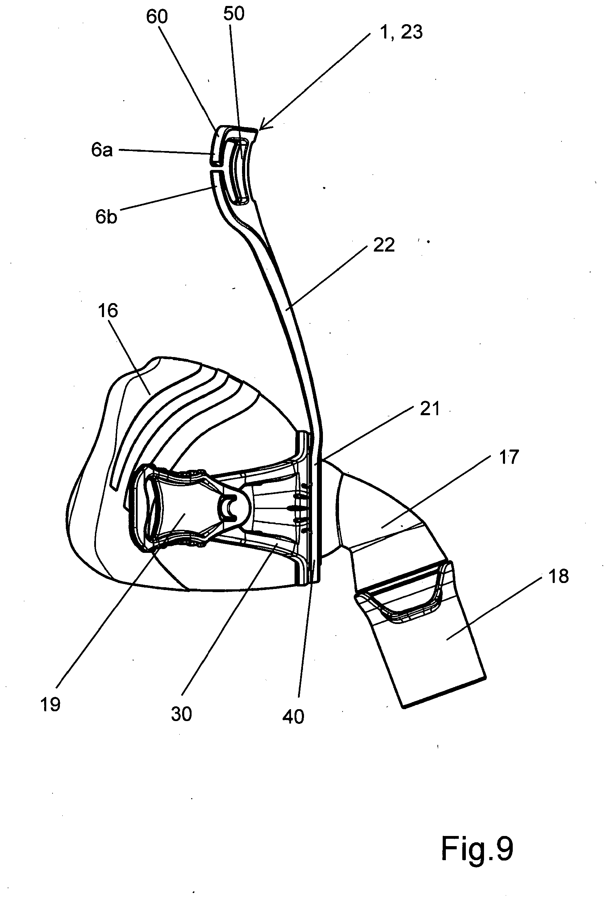

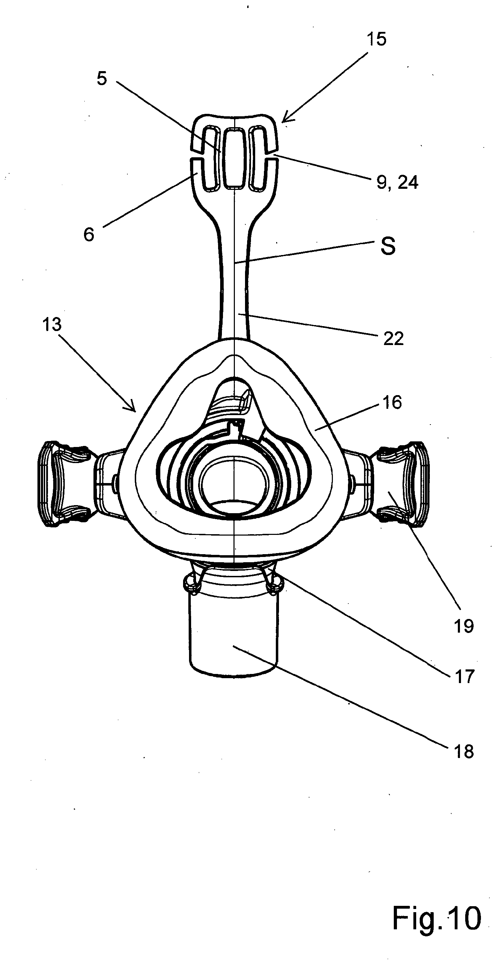

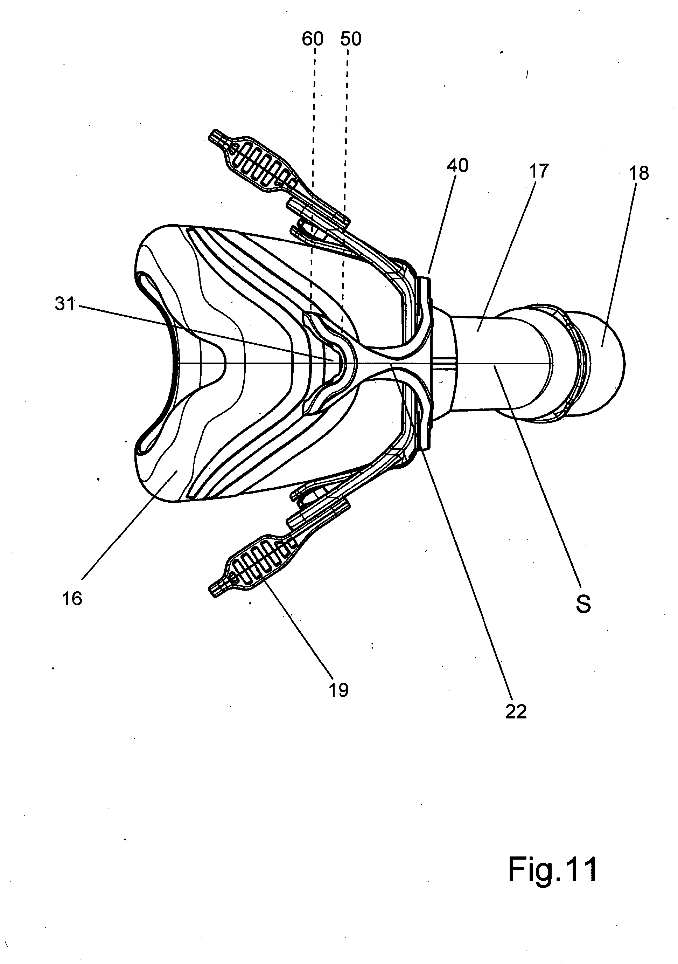

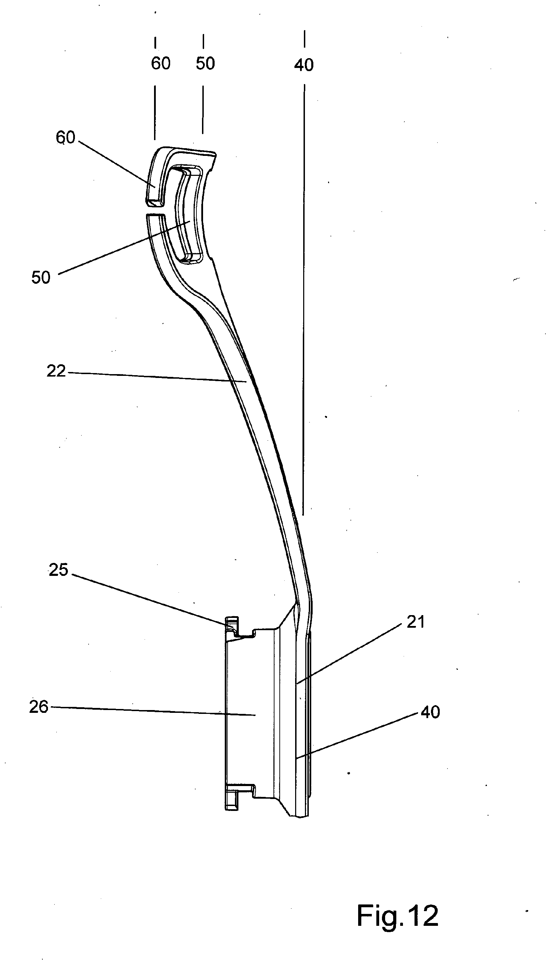

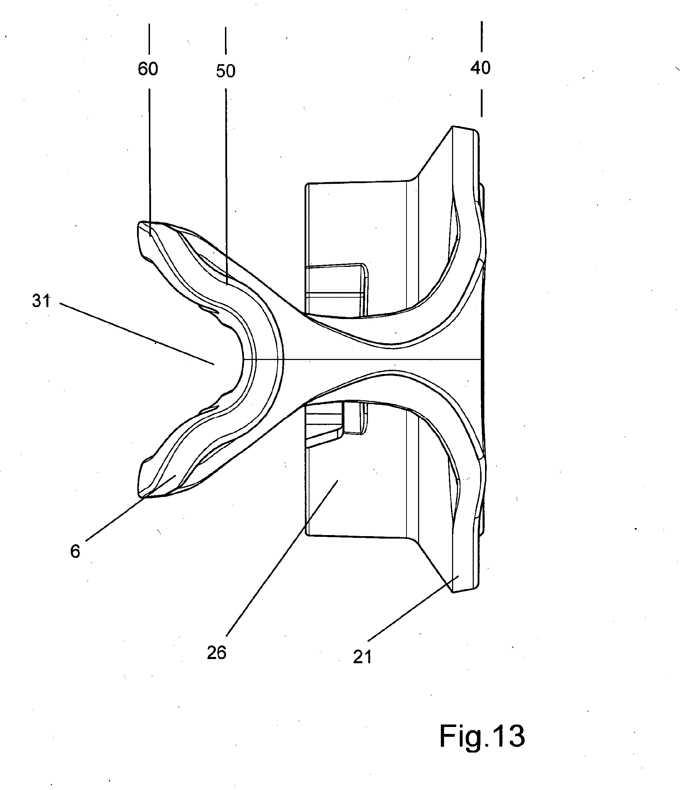

Die Fig. 9 bis 13 zeigen die erfindungsgemäße Atemmaske. Die erfindungsgemäße Atemmaske ist modular aufgebaut. Als Basis der Atemmaske (13) dient der Maskenkörper (14).Dieser ist beispielsweise als Spritzgussteil aus Kunststoff relativ fest ausgeführt. An dem Maskenkörper (14) setzen die Stirnstütze (15), die einen sicheren Sitz der Maske auf dem Gesicht eines nicht dargestellten Patienten gewährleistet, und der Maskenwulst (16), der durch seine Passform und seine elastische Beschaffenheit für einen dichten Abschluss der Atemmaske am Patienten sorgt, an. Das Anschlussstück (17) stellt die Verbindung zu einem nicht dargestellten Schlauch dar, über den das Atemgas von einem Beatmungsapparat zur Atemmaske transportiert wird. Der Schlauch wird mittels einer auf dem Anschlussstück (17) drehbeweglich gelagerten Hülse (18) mit dem Anschlussstück (17) verbunden. Mithilfe von seitlich mit dem Maskenkörper (13) verbindbaren Clips (19) kann die Atemmaske durch Bänder sicher am Kopf des Patienten fixiert werden.

Zu erkennen ist die Symmetrie des Aufbaus entlang der vertikalen Mittelachse (s). An beiden Seiten des Maskenkörpers (13) befindet sich hier ein Clip (19) zur Bandaufnahme, jedoch ist auch eine asymmetrische Ausführungsform denkbar. Dies kann beispielsweise umgesetzt werden, indem auf einer Seite die Verbindung von Atemmaske und Halteband mit einem Clip (19) realisiert wird, während auf der anderen Seite eine Schlaufe und ein Haken genutzt werden.

Zu sehen ist eine trianguläre Grundform des Maskenwulstes (16) sowie das Innere der Atemmaske durch die patientenseitige Öffnung des Maskenwulstes hindurch. An der oberen Seite des Maskenwulstes (3) ragt die Stirnstütze (15) hervor. An den Seiten der Atemmaske sind die mit dem Maskenkörper (13) verbundenen Clips (19) zur Bandankopplung zu sehen. An der unteren Seite des Maskenwulstes (16) ragt das Anschlussstück (17) mit der montierten Hülse (18) hervor.

In einer Ansicht ist eine der menschlichen Anatomie angepasste Formgebung der Stirnstütze (15) erkennbar. Aus der sich aus der Verbindung mit dem Maskenkörper (13) ergebenden Fläche ragt das Kopfende der Stirnstütze (15) in Richtung eines Patienten, um auch ohne übermäßiges Neigen der gesamten Atemmaske eine ausreichende Stützfunktion an der Stirn eines Patienten zu erreichen.

Die Basis der Stirnstütze (13) bildet der Stirnstützenverbinder (21), mit dem die Stirnstütze (13) am Maskenkörper befestigt wird. Ein Steg (22) verbindet den Stirnstützenverbinder (21) mit der Bandankopplung (1, 23) am Kopf- der Stirnstütze. Die Bandankopplung (23) besteht aus drei horizontal nebeneinander angeordneten, in vertikaler Richtung länglich ausgedehnten Aussparungen in der Struktur der Stirnstütze. Die beiden äußeren Aussparungen weisen jeweils einen zusätzlichen Spalt (9,24) in der nach außen gewandten Struktur der Stirnstütze auf. Diese Spalte (24) können zum Einfädeln der Haltebänder auf Stirnhöhe eines Patienten in die Bandankopplung (23) genutzt werden. Alternativ kann das Band auch durch die mittig positionierte Aussparung geführt werden.

Es ist auch an andere Ausführungsformen der Bandankopplung (23) gedacht. Beispielsweise kann diese auch nur durch zwei Aussparungen mit zusätzlichem Spalt (24) umgesetzt werden.

Der Steg (22) ist in seiner Breite in der gezeigten Ausführungsform gegenüber dem Kopfteil mit Bandankopplung (23) und dem Stirnstützenverbinder (21) signifikant reduziert. Dies ermöglicht ein ansprechendes, schlankes Design und die Einsparung von Material.

Der Stirnstützenverbinder (21) bietet zur Vervollständigung des im Maskenkörper (1) angelegten Bajonettverschlusses eine in Positionierung und Anzahl der im Maskenkörper vorhandenen Kanäle entsprechende Anzahl an Verschlusszähnen (25). Diese stehen auf der äußeren Kontur des Verbindungsringes (26) radial nach außen und weisen ihrerseits jeweils einen weiteren radial nach außen gerichteten Zahn auf, der gemeinsam mit dem Rastzahn im Maskenkörper für die Arretierung von Maskenkörper (30) und Stirnstütze (13) in der vorgesehenen Position sorgt. Die innere Kontur des Stirnstützenverbinders (21) ist als Kugelkäfig (27) ausgeführt, der das Anschlussstück (17) beweglich lagern kann. Zum Anschlussstück (17) hin wird der Kugelkäfig (27) durch eine sich nach außen verengende Ringstruktur (28) begrenzt.

Zu erkennen ist, dass das Kopfteil der Stirnstütze (13) in Relation zum Stirnstützenverbinder (21) zu einem Patienten hin ragt. Die Form des die Bandankopplung (23) beherbergenden Kopfes der Stirnstütze (2) weist eine Aussparung (31) auf, die zwischen der Stirn eines Patienten und der Bandankopplung (23) in der Stirnstütze (2) einen Raum bietet. Die Aussparung (31) weist gemeinsam mit den Strukturelementen der Bänderungsankopplung, in einer Ansicht von oben eine Y-Form auf. Der Konturverlauf im Bereich der Aussparung (31) verläuft gerundet mit einem oder mehreren Radien. Entsprechend des Materials der Bänderungsankopplung und der Wandstärken kann die Y-Struktur eine Elastizität bieten, die eine Adaptation des Kopfteils der Stirnstütze (13) relativ zur Stirn des Patienten erlaubt.

Bei einer rotationssymmetrischen Ausführung des Maskenkörpers (30) kann dieser in genau zwei um 180° verschobenen Positionen mit der Stirnstütze (13) verbunden werden, die funktional identisch sind. Damit lässt sich eine Vereinfachung der Montage der Module der Atemmaske erreichen.

Das Anschlussstück (17) ist als abgewinkelte Rohrstruktur ausgeführt und weist an einem Ende eine Oberflächenkontur (32) auf, die einer Teilkugel entspricht. Diese Teilkugel (32) dient der beweglichen Verbindung von Anschlussstück (17) und Stirnstütze (13) mittels des im Stirnstützenverbinder (21) realisierten Kugelkäfigs (27).

Am anderen Ende ist eine drehbar gelagerte Hülse (18) als Anschluss für einen nicht dargestellten Schlauch montiert.

Der Stirnstützenverbinder (21) definiert eine erste Ebene (40). Im Bereich der Bänderungsankopplung der Stirnstütze wird eine zweite Ebene (50) durch die Aufnahmemittel 5 gebildet. Im Bereich der Bänderungsankopplung der Stirnstütze wird auch eine dritte Ebene (60) durch die Aufnahmemittel 6 gebildet.

Die Ebenen sind im Wesentlichen parallel zueinander angeordnet. Dabei ist die erste Ebene (40) weiter von der dritten Ebene (60) beabstandet als von der zweiten Ebene (50). Der Versatz von der ersten Ebene (40) zu der zweiten Ebene (50) beträgt 1 cm bis 4 cm, bevorzugt 1,2 cm bis 3 cm. Der Versatz von der zweiten Ebene (50) zu der dritten Ebene (60) beträgt 3 mm bis 23 mm, bevorzugt 4 mm bis 15 mm.

Die Ebenen sind alternativ in einem Winkel zueinander angeordnet. Dabei ist zweiten Ebene (50) in einem Winkel von 1° bis 20° zur ersten Ebene (40) in Richtung auf die Stirn eines Patienten geneigt. Alternativ oder ergänzend ist die dritte Ebene (60) in einem Winkel von 1° bis 20° zur ersten Ebene (40) in Richtung auf die Stirn eines Patienten geneigt. Alternativ oder ergänzend ist die dritte Ebene (60) in einem Winkel von 1° bis 20° zur zweiten Ebene (40) geneigt.

## Patentansprüche

1. Patienteninterface mit einem Kopplungselement 1 für eine Bänderung das einstückig als Teil einer Stirnabstützung des Patienteninterface ausgebildet ist , wobei das Kopplungselement 1 zumindest eine Symmetrieebene S aufweist und zumindest vier Aufnahmemittel 5, 6 für ein Bänderungsende 4 einer Bänderung 3 wobei die Aufnahmemittel 5, 6 in Paaren beidseitig der Symmetrieebene S angeordnet sind und ein Bänderungsende 4 alternativ an dem Aufnahmemittel 5 oder an dem Aufnahmemittel 6 auf einer Seite des Kopplungselementes befestigbar ist und das Aufnahmemittel 5 räumlich benachbart zum Aufnahmemittel 6 und näher zu der Symmetrieebene S angeordnet ist als das Aufnahmemittel 6, wobei das Kopplungselement 1 zumindest eine Ebene (50) aufweist, die parallel zu der Anlagefläche, nämlich der Stirn eines Benutzers, verläuft, wobei das Kopplungselement 1 sich zumindest abschnittsweise abgewinkelt von der Ebene mit einer gerundeten Kontur erstreckt und eine Aussparung (31) definiert und wobei die Aufnahmemittel 5, 6 unterschiedlich weit von der Stirn eines Benutzers entfernt angeordnet sind,
wobei die Aufnahmemittel 5, 6 als geschlossener oder geschlitzter Steg ausgebildet sind, und
wobei die geschlossenen Stege 5 weiter innen, relativ zu den geschlitzten Stegen 6 außen, näher an der Symmetrieebene S angeordnet sind,
**dadurch gekennzeichnet, dass** die geschlitzten Stege 6 und/oder die geschlossenen Stege 5 windschief zueinander und/oder zur Symmetrieebene S verlaufen.

2. Patienteninterface nach zumindest einem der vorhergehenden Ansprüche **dadurch gekennzeichnet, dass** durch die Aufnahmemittel 5 eine Ebene (50) gebildet ist und durch die Aufnahmemittel 6 eine weitere Ebene (60) gebildet wird, wobei die Ebenen (50, 60) voneinander beabstandet sind.

3. Patienteninterface nach zumindest einem der vorhergehenden Ansprüche **dadurch gekennzeichnet, dass** die Ebenen (50, 60) parallel zueinander oder in einem Winkel zueinander verlaufen.

4. Patienteninterface nach zumindest einem der vorhergehenden Ansprüche **dadurch gekennzeichnet, dass** durch die Aufnahmemittel 5 und/oder die Aufnahmemittel 6 zumindest abschnittsweise einen gebogenen Konturverlauf aufweisen.

5. Patienteninterface nach zumindest einem der vorhergehenden Ansprüche **dadurch gekennzeichnet, dass** der gebogene Konturverlauf, von einem mittleren Bereich des Ausnahmemittels ausgehend, einen ansteigenden Krümmungsradius aufweist.

6. Patienteninterface nach zumindest einem der vorhergehenden Ansprüche **dadurch gekennzeichnet, dass** die Aufnahmemittel 5 und 6 geometrisch unterschiedlich ausgebildet sind.

7. Patienteninterface nach zumindest einem der vorhergehenden Ansprüche **dadurch gekennzeichnet, dass** die Aufnahmemittel als geschlossener 5 oder geschlitzter Steg 6 ausgebildet sind.

8. Patienteninterface nach zumindest einem der vorhergehenden Ansprüche **dadurch gekennzeichnet, dass** das Kopplungselement 2 einstückig als Teil einer Stirnabstützung eines Patienteninterface ausgebildet ist und die Symmetrieebene S mit der Längsachse der Stirnabstützung zusammenfällt.

9. Patienteninterface nach zumindest Anspruch 7 **dadurch gekennzeichnet, dass** die geschlitzten Stegen 6 außen und die geschlossenen Stege 5 relativ zu den geschlitzten Stegen weiter innen, näher an der Symmetrieebene S angeordnet sind.

10. Patienteninterface nach zumindest einem der Ansprüche 7 oder 9 **dadurch gekennzeichnet, dass** die geschlitzten Stege 6 und die geschlossenen Stege 5 zumindest abschnittsweise parallel zueinander verlaufen.

11. Patienteninterface nach zumindest einem der Ansprüche 7, 9, 10 oder 11 **dadurch gekennzeichnet, dass** ein geschlitzter Steg 6 zwischen den beiden Teilstegen 6a, 6b jeweils eine Öffnung 9 für die Bänderung bereitstellt und Öffnung so dimensioniert ist, dass die Bänderung mit ihrer flachen Seite hindurchgefädelt werden kann.

12. Patienteninterface nach zumindest einem der vorhergehenden Ansprüche **dadurch gekennzeichnet, dass** im Bereich des Patienteninterface eine erste Ebene (40) im Bereich des Maskenkörpers oder der Basis der Stirnstütze definiert ist, im Bereich der Bänderungsankopplung der Stirnstütze Aufnahmemittel 5 eine zweite Ebene (50) gebildet ist und im Bereich der Bänderungsankopplung der Stirnstütze durch die Aufnahmemittel 6 eine dritte Ebene (60) gebildet wird.

13. Patienteninterface nach zumindest einem der vorhergehenden Ansprüche **dadurch gekennzeichnet, dass** die erste Ebene (40) weiter von der dritten Ebene (60) beabstandet ist als von der zweiten Ebene (50).

## Claims

1. A patient interface comprising a coupling element 1 for a strap, which coupling element is designed as an integral part of a forehead support of the patient interface, the coupling element 1 comprising at least one plane of symmetry S and at least four receiving means 5, 6 for a strap end 4 of a strap 3, the receiving means 5, 6 being arranged in pairs on both sides of the plane of symmetry S and it being possible to fasten a strap end 4 on one side of the coupling element either to the receiving means 5 or to the receiving means 6 and the receiving means 5 being arranged spatially adjacent to the receiving means 6 and closer to the plane of symmetry S than the receiving means 6, the coupling element 1 comprising at least one plane (50) that extends in parallel with the contact surface, namely the forehead of a user, the coupling element 1 extending at least in portions at an angle from the plane with a rounded contour and defining a recess (31) and the receiving means 5, 6 being arranged at different distances from the forehead of a user, the receiving means 5, 6 being designed as a closed or slotted rib, and the closed ribs 5 being arranged further in', relative to the slotted ribs 6 on the outside, closer to the plane of symmetry s, **characterized in that** the slotted ribs 6 and/or the closed ribs 5 extend askew relative to one another and/or to the axis of symmetry S.

2. The patient interface according to at least one of the preceding claims, **characterized in that** a plane (50) is formed by the receiving means 5 and another plane (60) is formed by the receiving means 6, the planes (50, 60) being spaced apart from one another.

3. The patient interface according to at least one of the preceding claims, **characterized in that** the planes (50, 60) extend in parallel with one another or at an angle to one another.

4. The patient interface according to at least one of the preceding claims, **characterized in that** the receiving means 5 and/or the receiving means 6 have a curved contour shape at least in portions.

5. The patient interface according to at least one of the preceding claims, **characterized in that** the curved contour shape has an increasing radius of curvature, proceeding from a central region of the receiving means.

6. The patient interface according to at least one of the preceding claims, **characterized in that** the receiving means 5 and 6 are designed to be geometrically different.

7. The patient interface according to at least one of the preceding claims, **characterized in that** the receiving means are designed as a closed 5 or slotted rib 6.

8. The patient interface according to at least one of the preceding claims, **characterized in that** the coupling element 2 is designed as an integral part of a forehead support of a patient interface and the plane of symmetry S coincides with the longitudinal axis of the forehead support.

9. The patient interface according to at least claim 7, **characterized in that** the slotted ribs 6 are arranged on the outside and the closed ribs 5 are arranged further in, relative to the slotted ribs, closer to the plane of symmetry S.

10. The patient interface according to at least one of claims 7 or 9, **characterized in that** the slotted ribs 6 and the closed ribs 5 extend in parallel with one another at least in portions.

11. The patient interface according to at least one of claims 7, 9, 10 or 11, **characterized in that** a slotted rib 6 provides an opening 9 for the strap between the two partial ribs 6a, 6b in each case and the opening is dimensioned such that the strap can be threaded through with the flat side thereof.

12. The patient interface according to at least one of the preceding claims, characterized -in that, in the region of the patient interface, a first plane (40) is defined in the region of the mask body or base of the forehead support, a second plane (50) is formed in the region of the strap coupling of the forehead support by means of the receiving means 5, and a third plane (60) is formed in the region of the strap coupling of the forehead support by means of the receiving means 6.

13. The patient interface according to at least one of the preceding claims, **characterized in that** the first plane (40) is spaced further apart from the third plane (60) than from the second plane (50).

## Revendications

1. Interface patient comprenant un élément d'accouplement (1) pour un harnais, lequel est conçu intégralement comme une pièce d'un appui frontal de l'interface patient, dans laquelle l'élément d'accouplement (1) présente au moins un plan de symétrie (S) et au moins quatre moyens de réception (5, 6) pour une extrémité de harnais (4) d'un harnais (3), dans laquelle les moyens de réception (5, 6) sont disposés par paires des deux côtés du plan de symétrie (S) et une extrémité de harnais (4) peut être fixée alternativement au moyen de réception (5) ou au moyen de réception (6) sur un côté de l'élément d'accouplement, et le moyen de réception (5) est disposé spatialement à proximité du moyen de réception (6) et plus près du plan de symétrie (S) que le moyen de réception (6), dans laquelle l'élément d'accouplement (1) présente au moins un plan (50) s'étendant parallèlement à la surface de contact, notamment le front d'un utilisateur, dans laquelle l'élément d'accouplement (1) s'étend au moins partiellement de façon coudée par rapport au plan avec un contour arrondi et définit un évidement (31) et dans laquelle les moyens de réception (5, 6) se trouvent à des distances différentes du front d'un utilisateur, dans laquelle les moyens de réception (5, 6) sont conçus comme une barrette fermée ou fendue, et dans laquelle les barrettes fermées (5) sont disposées plus à l'intérieur par rapport aux barrettes fendues (6) à l'extérieur, plus près du plan de symétrie (S), **caractérisée en ce que** les barrettes fendues (6) et/ou les barrettes fermées (5) s'étendent de façon inclinée les unes par rapport aux autres et/ou par rapport à l'axe de symétrie (S) .

2. Interface patient selon l'une au moins des revendications précédentes, **caractérisée en ce que** les moyens de réception (5) forment un plan (50) et les moyens de réception (6) forment un autre plan (60), les plans (50, 60) étant espacés l'un de l'autre.

3. Interface patient selon l'une au moins des revendications précédentes, **caractérisée en ce que** les plans (50, 60) s'étendent parallèlement l'un à l'autre ou selon un angle l'un par rapport à l'autre.

4. Interface patient selon l'une au moins des revendications précédentes, **caractérisée en ce que** les moyens de réception (5) et/ou les moyens de réception (6) présentent au moins partiellement un contour courbe.

5. Interface patient selon l'une au moins des revendications précédentes, **caractérisée en ce que** le contour courbe présente un rayon de courbure croissant à partir d'une région médiane du moyen de réception.

6. Interface patient selon l'une au moins des revendications précédentes, **caractérisée en ce que** les moyens de réception (5 et 6) sont conçus différemment quant à leur géométrie.

7. Interface patient selon l'une au moins des revendications précédentes, **caractérisée en ce que** les moyens de réception sont conçus comme une barrette fermée (5) ou comme une barrette fendue (6).

8. Interface patient selon l'une au moins des revendications précédentes, **caractérisée en ce que** l'élément d'accouplement (2) est conçu intégralement comme une pièce d'un appui frontal d'une interface patient et le plan de symétrie (S) coïncide avec l'axe longitudinal de l'appui frontal.

9. Interface patient au moins selon la revendication 7, **caractérisée en ce que** les barrettes fendues (6) sont disposées à l'extérieur et les barrettes fermées (5) sont disposées plus à l'intérieur par rapport aux barrettes fendues, plus près du plan de symétrie (S).

10. Interface patient selon l'une au moins des revendications 7 et 9, **caractérisée en ce que** les barrettes fendues (6) et les barrettes fermées (5) s'étendent au moins partiellement parallèlement les unes aux autres.

11. Interface patient selon l'une au moins des revendications 7, 9, 10 et 11, **caractérisée en ce qu'**une barrette fendue (6) offre respectivement une ouverture (9) pour le harnais entre les deux barrettes partielles (6a, 6b), l'ouverture étant dimensionnée de manière à pouvoir insérer le harnais avec son côté plat à travers celle-ci.

12. Interface patient selon l'une au moins des revendications précédentes, **caractérisée en ce que** dans la région de l'interface patient, un premier plan (40) est défini dans la région du corps de masque ou de la base de l'appui frontal, un deuxième plan (50) est formé par les moyens de réception (5) dans la région de l'accouplement de harnais de l'appui frontal, et un troisième plan (60) est formé par les moyens de réception (6) dans la région de l'accouplement de harnais de l'appui frontal.

13. Interface patient selon l'une au moins des revendications précédentes, **caractérisée en ce que** le premier plan (40) est plus éloigné du troisième plan (60) que du deuxième plan (50).
